# EUROPEAN PATENT APPLICATION

(11) **EP 4 700 116 A1**
(43) Date of publication of application: **25.02.2026**
(21) Application number: 24792699.1
(22) Date of filing: 17.04.2024
(51) Int. Cl.: C12N 5/071, A61K 35/14, A61P 29/00, A61P 33/14, A61P 37/02, A61P 37/08, C07K 14/55, C12N 5/10

(54) **METHOD FOR PRODUCING TYPE 2 INNATE LYMPHOCYTES**

(30) Priority: 18.04.2023 JP 2023068042
(71) Applicant: Kyoto University, Kyoto-shi, Kyoto 606-8501 (JP); Astellas Pharma Inc., Tokyo 103-8411 (JP)
(72) Inventor: KANEKO, Shin, Kyoto-shi, Kyoto 606-8501 (JP); SUMIDE, Keisuke, Kyoto-shi, Kyoto 606-8501 (JP); NAGANO, Yuji, Tokyo 103-8411 (JP); KATO, Akemi, Tokyo 103-8411 (JP)
(74) Representative: Hoffmann Eitle
(86) International application number: PCT/JP2024/015294
(87) International publication number: WO 2024/219427

(57) **Abstract**

The present invention is directed to establishing a technique for producing group 2 innate lymphoid cells. Culturing hematopoietic precursor cells in the presence of CDK8 inhibitor enables to obtain group 2 innate lymphoid cells.

## Description

### TECHNICAL FIELD

The present invention relates to a technique for obtaining group 2 innate lymphoid cells, and relates especially to a technique for obtaining group 2 innate lymphoid cells from pluripotent stem cells.

### BACKGROUND ART

Innate lymphoid cells (ILCs) are a cell group of the innate immune system that have no antigen receptors, and are differentiated from common precursor cells and comparatively newly identified. ILCs have roles in rapidly producing cytokines characteristic of subsets in response to a stimulus from infected or injured tissue to cause immunoreaction including both innate immunity and acquired immunity, and control the directionality thereof. In general, ILCs are classified into five groups, NK cells, ILC1s, ILC2s, ILC3s, and LTi cells (lymphoid tissue inducer cells) by differences in produced cytokine and transcription factor that regulates the development and the function thereof.

Group 2 innate lymphoid cells (ILC2s), which are a type of ILCs, are resident in fat tissue and peripheral tissues in the lungs, the intestinal tract, the skin, and others. ILC2s are activated by signals due to "alarmins" such as IL-25, IL-33, and TSLP released upon the injury of epithelia to produce type 2 cytokines such as IL-5 and IL-13 in large amounts, resulting in activation of helper T2 cells (Th2 cells) or eosinophils. While ILC2s play an important role in initial defense against parasitic infection or tissue repair, it has been revealed that ILC2s are related to various diseases such as allergic diseases such as bronchial asthma and chronic rhinosinusitis accompanied by nasal polyps (CRSwNPs); inflammatory diseases; and autoimmune diseases. The transcription factor GATA3 is essential for developing ILC2s and maintaining the function thereof (NPLs 1 to 2).

It has been reported that IL-10-producing ILC2s referred to as ILCreg, ILC2reg, ILC2₁₀, and IL-10+ ILC2 are present as new subsets of ILC2s in human tissue and mouse tissue in which ILC2s are activated (NPLs 3 to 6). While it has been suggested that such ILC2 subsets are related to human allergic disease (NPL 7), it is known that the activation of ILC2s contributes to inflammatory subsidence as well as the repair of injured tissue.

The adoptive transfer of IL-10-producing ILC2s or the induction into IL-10-producing ILC2s *in vivo* expectedly establishes therapy for allergic diseases, immune diseases, and inflammatory diseases due to the antiinflammatory effect and immunosuppressive effect of IL-10 besides tissue repair and regenerative effect of ILC2s.

It has been reported that all the ILC subsets including NK cells, ILC1s, ILC2s, and ILC3s are *in vitro* produced from CD34-positive hematopoietic precursor cells derived from human cord blood and bone marrow, or ILC precursor cells in human adult peripheral blood (NPLs 8 to 9).

### CITATION LIST

### NON PATENT LITERATURE

NPL 1: Cell. 2018 Aug 23;174(5):1054-1066.
NPL 2: Nat Med. 2015 Jul; 21(7): 698-708.
NPL 3: Nat Commun. 2017 Dec 1;8(1):1900.
NPL 4: J Allergy Clin Immunol. 2019 Jun;143(6) :2190-2201.e9.
NPL 5: J Allergy Clin Immunol. 2021 Apr;147(4):1281-1295.e5.
NPL 6: J Exp Med. 2020 Feb 3;217(2):e20191520.
NPL 7: Immunity 2021; 54: 291-307.
NPL 8: Cell 2017; 168(6):1086-1100.
NPL 9: Immunity 2021; 54: 2417-2432.e5

### SUMMARY OF INVENTION

### TECHNICAL PROBLEM

Since ILC2s or ILC precursor cells are quantitatively limited that can be isolated from human peripheral blood and subjected to expansion culture, it is expectedly difficult to use these as a supply source for cell therapy.

For example, embryonic stem cells (ES cells) or induced pluripotent stem cells (iPS cells), obtained by introducing a reprograming factor into somatic cells, have been reported as cells with pluripotency. These cells can be almost infinitely proliferated while maintaining the pluripotency. If a method for inducing ILC2s from pluripotent stem cells could be developed, the method could be effective mean in the production of cell therapeutic drugs. The use of the induced ILC2s would also enable exploring or evaluating a drug for regulating the function of ILC2s related to disease or a drug for changing ILC2s in the living body into a subset such as IL-10-producing ILC2s having a therapeutic effect.

A method has ever been reported involving inducing NK cells/ILCs from iPS cells through CD34-positive hematopoietic precursor cells in order to apply ILCs to cellular immunotherapy for causing antitumor immunoreaction (Cancer Sci. 2020 May; 111(5): 1478-1490).

Unfortunately, any method for inducing ILC2s from iPS cells has never been reported.

The present invention is directed to developing a technique for producing ILC2s in view of such a situation.

### SOLUTION TO PROBLEM

In order to achieve the above-mentioned object, the present inventors have earnestly examined to find that hematopoietic precursor cells can be cultured in the presence of CDK8 inhibitor to prepare ILC2s.

The present invention includes, but not limited to, the following aspects.
[1] A method for producing group 2 innate lymphoid cells, including a step of culturing hematopoietic precursor cells in the presence of CDK8 inhibitor to obtain group 2 innate lymphoid cells.
[2] The method according to [1], wherein the group 2 innate lymphoid cells contain group 2 innate lymphoid cells that produce IL-10.
[3] The method according to [1] or [2] to be performed *in vitro.*
[4] The method according to [1] or [2], wherein the group 2 innate lymphoid cells are obtained by culturing hematopoietic precursor cells in the presence of CDK8 inhibitor, IL-2, and IL-33.
[5] The method according to [1] or [2], wherein the CDK8 inhibitor contains Senexin A, CCT-251921, MSC2530818, CCT251545, SEL120-34A, or BRD6989.
[6] The method according to [1] or [2], wherein the hematopoietic precursor cells are differentiated from human induced pluripotent stem cells (human iPS cells).
[7] The method according to [1] or [2], wherein the hematopoietic precursor cells are derived from the human body.
[8] The method according to [1] or [2], further including a step of inducing differentiation from pluripotent stem cells to the hematopoietic precursor cells.
[9] The method according to [8], wherein the pluripotent stem cells are induced pluripotent stem cells (iPS cells).
[10] The method according to [8], wherein the pluripotent stem cells are human iPS cells.

### ADVANTAGEOUS EFFECTS OF INVENTION

The present invention enables artificially obtaining ILC2s from hematopoietic precursor cells. In the present invention, hematopoietic precursor cells are also usable that are derived from pluripotent stem cells such as iPS cells. The present invention enables differentiation-inducing ILC2s from iPS cells or other cells. The present invention also enables obtaining ILC2s that produce IL-10 (IL-10-producing ILC2s). The present invention is effective in medicine development.

### BRIEF DESCRIPTION OF DRAWINGS

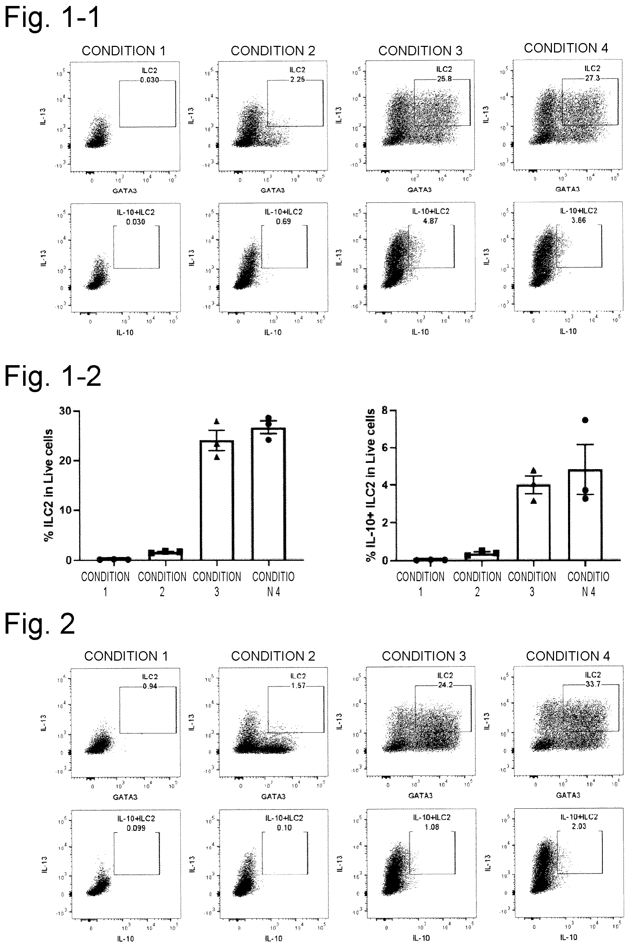

[Fig. 1-1] Fig. 1-1 is representative examples of figures confirming differentiation induction from hematopoietic precursor cells (derived from iPS cell line 1231A2) to ILC2s and IL-10-producing ILC2s (Experiment 2). The differentiation into ILC2s was confirmed by flow cytometry using the expression of GATA3 and the capacities to produce IL-13 and IL-10 as indices. The upper figures are graphs showing the expression levels of IL-13 and GATA3 in cells negative to lineage markers (CD3, CD14, CD16, CD19, CD20, and CD94) (ordinate: expression level of IL-13, abscissa: expression level of GATA3). The lower figures are graphs showing the expression levels of IL-13 and IL-10 in cells negative to the same lineage markers (ordinate: expression level of IL-13, abscissa: expression level of IL-10).
[Fig. 1-2] Fig. 1-2 is graphs showing the percentages of ILC2s and IL-10-producing ILC2s obtained by the same analysis as in Fig. 1-1 (n = 3, triplicate) (Experiment 2). The left figure shows the percentage of the ILC2s (cells that are negative to the lineage markers, positive to IL-13, and positive to GATA3) in the live cells. The right figure shows the percentage of the IL-10-producing ILC2s (cells that are negative to the lineage markers, positive to IL-13, and positive to IL-10) in the live cells. The error bars show ± standard errors from the means.
[Fig. 2] Fig. 2 is representative examples of figures confirming the differentiation induction from hematopoietic precursor cells (derived from iPS cell line Ff-I01s04) to ILC2s and IL-10-producing ILC2s (Experiment 3). The differentiation into ILC2s was confirmed by flow cytometry using the expression of GATA3 and the capacities to produce IL-13 and IL-10 as indices. The upper figures are graphs showing the expression levels of IL-13 and GATA3 in cells negative to the lineage markers (CD3, CD14, CD16, CD19, CD20, and CD94) (ordinate: expression level of IL-13, abscissa: expression level of GATA3). The lower figures are graphs showing the expression levels of IL-13 and IL-10 in cells negative to the same lineage markers (ordinate: expression level of IL-13, abscissa: expression level of IL-10).

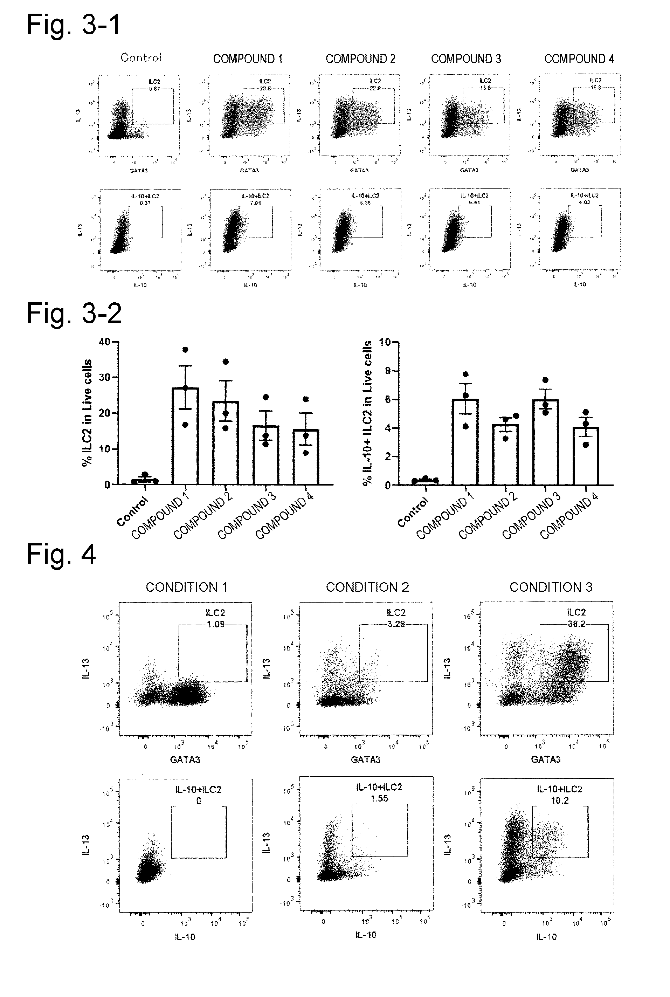

[Fig. 3-1] Fig. 3-1 is representative examples of figures confirming differentiation induction from hematopoietic precursor cells (derived from iPS cell line 1231A2) to ILC2s and IL-10-producing ILC2s using multiple CDK8 inhibitors having different structures (Experiment 4). The differentiation into ILC2s was confirmed by flow cytometry using the expression of GATA3 and the capacities to produce IL-13 and IL-10 as indices. The upper figures are graphs showing the expression levels of IL-13 and GATA3 in cells negative to the lineage markers (CD3, CD14, CD16, CD19, CD20, and CD94) (ordinate: expression level of IL-13, abscissa: expression level of GATA3). The lower figures are graphs showing the expression levels of IL-13 and IL-10 in cells negative to the same lineage markers (ordinate: expression level of IL-13, abscissa: expression level of IL-10).
[Fig. 3-2] Fig. 3-2 is graphs showing the percentages of ILC2s and IL-10-producing ILC2s obtained by the same analysis as in Fig. 3-1 (n = 3, triplicate), respectively (Experiment 4). The left figure shows the percentage of the ILC2s (cells that are negative to the lineage markers, positive to IL-13, and positive to GATA3) in the live cells. The right figure shows the percentage of the IL-10-producing ILC2s (cells that are negative to the lineage markers, positive to IL-13, and positive to IL-10) in the live cells. The error bars show ± standard errors from the means.
[Fig. 4] Fig. 4 is representative examples of figures confirming the differentiation induction from human cord blood-derived CD34-positive cells to ILC2s and IL-10-producing ILC2s (Experiment 5). The differentiation into ILC2s was confirmed by flow cytometry using the expression of GATA3 and the capacities to produce IL-13 and IL-10 as indices. The upper figures are graphs showing the expression levels of IL-13 and GATA3 in cells negative to the lineage markers (CD3, CD14, CD16, CD19, CD20, and CD94) (ordinate: expression level of IL-13, abscissa: expression level of GATA3). The lower figures are graphs showing the expression levels of IL-13 and IL-10 in cells negative to the same lineage markers (ordinate: expression level of IL-13, abscissa: expression level of IL-10).

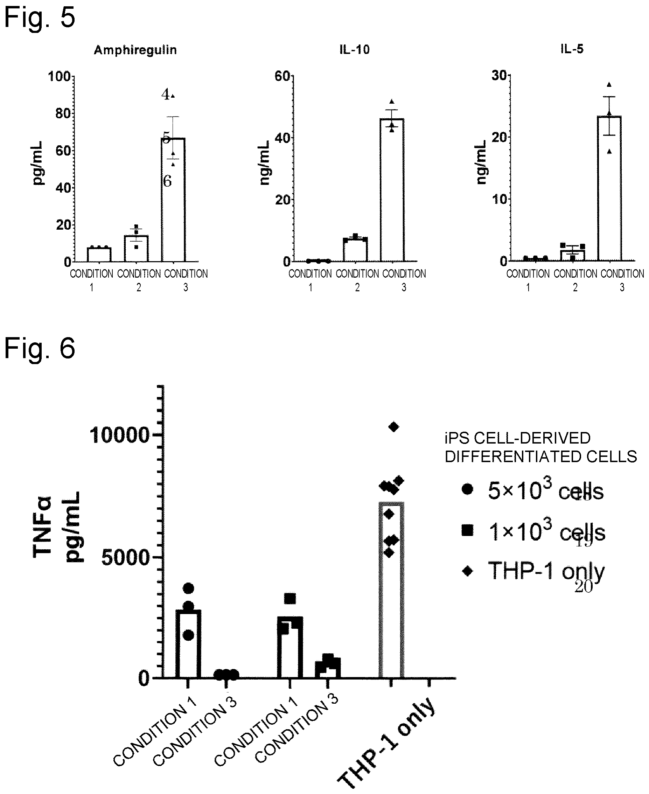

[Fig. 5] Fig. 5 is the results of the quantitative evaluation of the concentrations of cytokines in the differentiation culture supernatants of iPS cells-derived ILC2s (Experiment 6). Fig. 5 shows the concentration of amphiregulin (pg/mL), the concentration of IL-10 (ng/mL), and the concentration of IL-5 (ng/mL) from the left. The plots under the conditions show the concentrations of the cytokines in the differentiation culture supernatants of ILC2s derived from iPS cells of different lots, respectively. The error bars show ± standard errors from the means.
[Fig. 6] Fig. 6 is the results of quantitative evaluation of suppressive effects of differentiated cells containing IL-10-producing ILC2s on the production of TNFα by THP-1 cells (Experiment 7). The ordinate shows the concentration (pg/mL) of TNFα contained in the supernatant derived from the THP-1. With respect to Condition 1 and Condition 3, the values of three wells are shown. With respect to the THP-1 only group, the values of nine wells are shown.

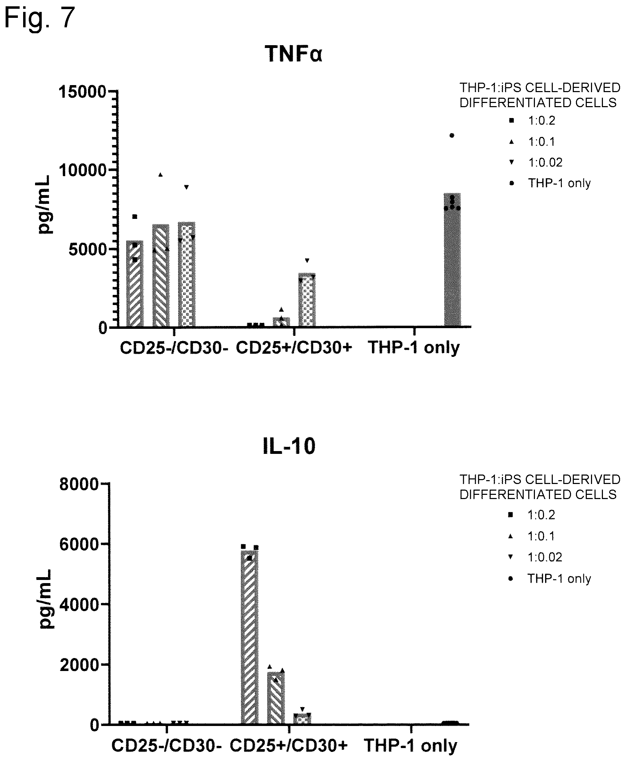

[Fig. 7] Fig. 7 is the results of quantitative evaluation of suppressive effects of a CD25-CD30- fraction and a CD25+CD30+ fraction separated from differentiated cells containing IL-10-producing ILC2s on the production of TNFα by THP-1 cells (Experiment 8). The ordinate of the upper figure shows the concentrations (pg/mL) of TNFα contained in the supernatants derived from THP-1. The ordinate of the lower figure shows the concentrations (pg/mL) of IL-10 produced by differentiated cells derived from iPS cells. With respect to the CD25-CD30- group and the CD25+CD30+ group, the values of three wells are shown. With respect to the THP-1 only group, the values of six wells are shown.

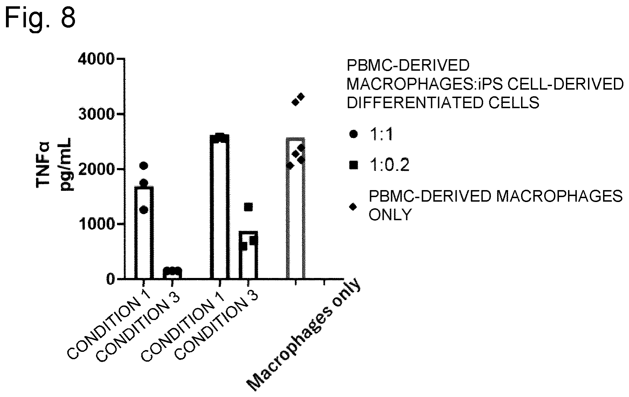

[Fig. 8] Fig. 8 is the results of quantitative evaluation of suppressive effects of differentiated cells containing IL-10-producing ILC2s on the production of TNFα by macrophages derived from human peripheral blood monocytes (PBMCs) (Experiment 9). The ordinate shows the concentration (pg/mL) of TNFα contained in the supernatants derived from the macrophages derived from the human PBMCs. With respect to Condition 1 and Condition 3, the values of three wells are shown. With respect to the PBMC-derived macrophages only group, the values of six wells are shown.

### DESCRIPTION OF EMBODIMENTS

The present invention relates to a method for obtaining group 2 innate lymphoid cells from hematopoietic precursor cells. The present invention includes group 2 innate lymphoid cells produced by the method according to the present invention and the use thereof.

The present invention specifically includes the following method:
A method for producing group 2 innate lymphoid cells, including a step of culturing hematopoietic precursor cells in the presence of CDK8 inhibitor to obtain group 2 innate lymphoid cells.

Although a technique for artificially obtaining group 2 innate lymphoid cells (ILC2s) from pluripotent stem cells has never been established, the present invention has established a technique for producing ILC2s. It has been found that ILC2s, which are cells of the innate immune system, are activated through no antigen recognition mechanism to produce a large amount of group 2 cytokine, and ILC2s are related to not only allergic inflammation but also pathological conditions of obesity, chronic inflammation such as fibrosis, and autoimmune disease such as rheumatism.

Although the ILC2s according to the present invention are not particularly limited, in the present invention, the confirmation of expression of GATA3 enables ascertaining that ILC2s are obtained from hematopoietic precursor cells for the following reason: The transcription factor GATA3 is essential for developing ILC2s and maintaining the function thereof. Since it is known that ILC2s produce IL-13 and IL-5 in large amounts, the confirmation of the capacity to produce IL-13 and the capacity to produce IL-5 enables ascertaining the presence of ILC2s. Furthermore, the differentiation from hematopoietic precursor cells to ILC2s can also be confirmed by ascertaining no expression of lineage markers such as CD3, CD4, CD16, CD19, CD20, and CD94.

ILC2s can be confirmed with the markers by various known methods. ILC2s can be confirmed by such as cytological staining using antibodies to these proteins or expression analysis using the quantitative PCR and the scRNA-seq of mRNAs thereof.

The presence of IL-10-producing ILC2s referred to as ILCreg or the like has been reported as a subset of ILC2s. IL-10 is known as a cytokine having the effect of suppressing inflammation. IL-10-producing ILC2s are considered to be particularly preferable in use for pharmaceutical purposes. In an embodiment, the ILC2s produced in the present invention contain an IL-10-producing ILC2 subset. The presence of the IL-10-producing ILC2s can be confirmed by ascertaining the capacity to producing IL-10. In an embodiment, the method of the present invention further includes a step of selecting IL-10-producing group 2 innate lymphoid cells (ILC2s) from the obtained ILC2.

ILC2s are selected with markers by various known methods. For example, if an antibody is used that specifically binds to a marker, examples include a method involving using a cell sorter with a fluorescence-labeled antibody (for example, FACS (registered trademark, BD Biosciences)), a method for magnetically selecting cells with antibody-labeled magnetic beads (for example, MACS (registered trademark, Miltenyi Biotec)), and a method using an antibody-immobilized support (for example, cell enrichment column). Antibodies are marketed that specifically bind to antigens. Such antibodies can be suitably used.

In the present invention, the hematopoietic precursor cells can be differentiated into hemocytes such as lymphoid cells, eosinophils, neutrophils, basophils, erythrocytes, and megakaryocytes. Examples of usable cell include all of the hematopoietic precursor cells collected directly from an individual organism; the primary cultured cells/the subcultured cells, the hematopoietic precursor cells being cultured and proliferated *in vitro* to obtain the primary cultured cells/the subcultured cells; the hematopoietic precursor cells differentiation-induced from pluripotent stem cells; and the unfractionated cell populations that contain these and can be hematopoietically differentiated. In an embodiment, the hematopoietic precursor cells used in the present invention are differentiation-induced from pluripotent stem cells (preferably induced pluripotent stem cells (iPS cells), more preferably human iPS cells). The pluripotent stem cells in the present invention are stem cells that have not only pluripotency that enables differentiating the stem cells into many types of cells in the living body having different properties and shapes but also proliferative capacity. The pluripotent stem cells in the present invention include any cells that can be induced into hematopoietic precursor cells. Examples of the pluripotent stem cells include, but not limited to, embryonic stem cells (ES cells), embryonic stem cells prepared by nuclear transfer technique (nuclear transfer embryonic stem cells: ntES cells), spermatogonial stem cells (germline stem cells: GS cells), embryonic germ cells (EG cells), induced pluripotent stem cells (iPS cells), and pluripotent cells derived from cultured fibroblasts or bone marrow stem cells (Multi-lineage differentiating stress enduring cells: Muse cells). Since iPS cells can be obtained without the disruption of embryos or ova, the pluripotent stem cells are preferably iPS cells, more preferably human iPS cells.

The method for producing iPS cells is known. iPS cells can be produced by introducing a reprograming factor into any somatic cells. Examples of the reprograming factor include genes of OCT3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3, and Glis1; or gene products thereof. These reprograming factors may be used alone or in combination.

The somatic cells include, but not limited to, all of the somatic cells of a neonate (baby) and the somatic cells of a healthy person or a patient, and include all of the primary cultured cells, the subcultured cells, and the cell lines derived therefrom. Specific examples of the somatic cells include (1) tissue stem cells (somatic stem cells) such as neural stem cells, hematopoietic stem cells, mesenchymal stem cells, and dental pulp stem cells; (2) tissue precursor cells; and (3) differentiated cells present in organs and tissues such as blood cells (including peripheral blood cells and cord blood cells), muscle cells, skin cells, hair cells, liver cells, gastric mucosal cells, enterocytes, splenocytes, pancreatic cells, brain cells, pneumocytes, renal cells, and adipocytes.

If iPS cells are used as a raw material of cells for transplantation, it is preferable from the viewpoint that rejection does not occur to use somatic cells having a human leukocyte antigen genotype identical or substantially identical with that of a recipient. The phrase "substantially identical" means that the HLA genotypes are so identical that an immunosuppressant can suppress immunoreaction on transplanted cells. Examples include somatic cells having identical HLA types in three genetic loci, HLA-A, HAL-B, and HAL-DR, or four genetic loci, HLA-A, HAL-B, HAL-DR, and HAL-C. Examples of the pluripotent stem cells usable as the raw material to be induced into hematopoietic stem cells include pluripotent stem cells that are prepared by the methods described in Nat. Biotechnol., (2017), 35: 765-772 and Nat Biomed Eng. 2021 May;5(5):429-440., and do not cause rejection in allogeneic transplantation. The pluripotent stem cells that do not cause rejection in allogeneic transplantation are preferably ES cells or iPS cells that do not cause rejection in allogeneic transplantation, more preferably human ES cells or human iPS cells that do not cause rejection in allogeneic transplantation.

In an embodiment, the hematopoietic precursor cells used in the present invention are derived from the human body. The hematopoietic precursor cells derived from the human body include all of the hematopoietic precursor cells collected directly from a human; the primary cultured cells/the subcultured cells, the hematopoietic precursor cells collected directly from a human cultured and proliferated *in vitro* to obtain the primary cultured cells/the subcultured cells; and unfractionated cell populations that contain these and can be hematopoietically differentiated. The human hematopoietic precursor cells can be collected, for example, from human cord blood or bone marrow. In an embodiment, the hematopoietic precursor cells derived from the human body are derived from human cord blood or bone marrow.

The culture method used in the present invention is not particularly limited. The culture may be performed by such as suspension culture and adherent culture.

Suspension culture means that cells are cultured without adhering to a culture dish. The suspension culture is not particularly limited. The culture can be performed with a culture dish not subjected to artificial treatment for improving the adhesiveness to cells (for example, treatment involving coating with the extracellular matrix) or a culture dish subjected to artificial treatment for suppressing the adherence.

Adherent culture means that cells are cultured while adhering to a culture dish. The adherent culture may be any adherent culture. The culture can also be performed on a culture dish subjected to coating treatment. Examples of the coating agent include Matrigel, collagen, gelatin, laminin, heparan sulfate proteoglycan, and entactin.

In the present invention, the culture temperature is not particularly limited. The culture is performed, for example, at around 30 to 40°C, preferably around 37°C, in a CO₂-containing air atmosphere. The concentration of CO₂ is around 2 to 5%, preferably around 5%. The culture time is not particularly limited. The culture is performed, for example, for 0.5 to 30 days, for 1 to 20 days, or for 3 to 10 days.

The medium may be prepared by a known method. For example, one or more substances can be suitably added to minimal essential medium for culturing animal cells to prepare the minimal essential medium. Examples of the minimal essential medium include IMDM (Iscove's modified Dulbecco's medium), Medium 199, EMEM (Eagle's minimum essential medium), αMEM (alpha modified Eagle minimum essential medium), DMEM (Dulbecco's modified Eagle's Medium), F12 medium (Ham's F12 medium),
RPMI1640 medium, Fischer's medium, and mixed media thereof. The medium may contain serum such as fetal bovine serum (FBS), or may be serum-free. For example, one or more serum substitutes such as albumin may be added as needed. The medium can contain one or more substances such as transferrin, fatty acid, insulin, collagen precursor, trace elements, 2-mercaptoethanol, 3'-thiolglycerol, lipid, amino acids, L-glutamine, GlutaMAX (Invitrogen), non-essential amino acids (NEAAs), vitamins, growth factors, low-molecular weight compounds, antibiotics, antioxidants, pyruvic acid, buffer, inorganic salts.

### Differentiation induction from pluripotent stem cells to hematopoietic precursor cells

In the present invention, hematopoietic precursor cells obtained from pluripotent stem cells such as iPS cells are usable as the hematopoietic precursor cells. In the present invention, the method for differentiation induction into hematopoietic precursor cells is not particularly limited. The hematopoietic precursor cells can be obtained by any method, for example, the method described in literatures, Cancer Sci. 2020 May; 111(5): 1478-1490, Cells. 2023 Jan; 12(2): 321., Blood (2008) 111 (11): 5298-5306, and PLoS One. 2011;6(7):e22261. In an embodiment, the method for producing group 2 innate lymphoid cells of the present invention further include a step of inducing the differentiation from pluripotent stem cells, preferably induced pluripotent stem cells (iPS cells), more preferably human iPS cells, to hematopoietic precursor cells. In an aspect, the step of inducing the differentiation of the hematopoietic precursor cells from the pluripotent stem cells such as iPS cells includes culturing pluripotent stem cells in medium containing one or more proteins selected from the group consisting of BMP4, SCF, VEGF, FGF-2, TPO (thrombopoietin), and FLt3L. In an aspect, if the hematopoietic precursor cells are differentiation-induced from pluripotent stem cells such as iPS cells, examples include a method involving coculturing pluripotent stem cells with C3H10T1/2 cells, followed by coculturing with C3H10T1/2 cells in the presence of VEGF, FLT3L, and SCF to obtain a net-like structure, preparing hematopoietic precursor cells from the net-like structure. In this case, vitamins C may be further added, followed by culture. The "net-like structure" as used herein refers to three-dimensional pluripotent stem cell-derived saccular structure (structure having a space inside), the structure being formed of, for example, an endothelial cell population and containing hematopoietic precursor cells inside. Furthermore, pluripotent stem cells can be cultured on C3H10T1/2 cells in the presence of VEGF in accordance with the method described in Takayama N., et al. J Exp Med. 2817-2830 (2010) to obtain a net-like structure, preparing hematopoietic precursor cells from the net-like structure. Examples of the method for producing hematopoietic precursor cells from pluripotent stem cells include methods by embryoid body formation and cytokine addition (Chadwick et al. Blood 2003, 102: 906-15; Vijayaragavan et al. Cell Stem Cell 2009, 4: 248-62; and Saeki et al. Stem Cells 2009, 27: 59-67) or a method involving coculture with stromal cells derived from a different species (Niwa A et al. J Cell Physiol. 2009 Nov;221(2):367-77.), and a method using the combination of cytokine addition and a coating agent (Matrigel or laminin fragments) (WO2011/115308).

The hematopoietic precursor cells as used herein refers to cells that can be differentiated into hemocytes such as lymphoid cells, eosinophils, neutrophils, basophils, erythrocytes, and megakaryocytes. The hematopoietic precursor cells can be identified by, for example, confirming the cells are CD34-positive, or CD34-positive and CD43-positive, which CD34 and CD43 are surface antigen.

### Differentiation induction from hematopoietic precursor cells to group 2 innate lymphoid cells

In the present invention, the hematopoietic precursor cells are cultured in the presence of CDK8 inhibitor to obtain ILC2s. As long as the CDK8 inhibitor inhibits CDK8, the CDK8 inhibitor is not particularly limited. Examples include Senexin A, CCT-251921, MSC2530818, CCT251545, SEL120-34A, and BRD6989. The CDK8 inhibitor according to the present invention may inhibit CDK19, which is a homologue of CDK8. If Senexin A is used as the CDK8 inhibitor, the concentration thereof is preferably 0.01 to 1000 µM, more preferably 0.1 to 100 µM, further preferably 1 to 10 µM. If CCT-251921 is used as the CDK8 inhibitor, the concentration thereof is preferably 0.01 nM to 100 µM, more preferably 0.01 nM to 10 µM, further preferably 10 nM to 1 µM. If MSC2530818, CCT251545, and SEL120-34A are used, each of the concentrations thereof is preferably 0.01 nM to 100 µM, more preferably 0.1 to 100 µM, further preferably 0.3 to 10 µM. If BRD6989 is used, the concentration thereof is preferably 0.01 nM to 100 µM, more preferably 0.1 to 100 µM, further preferably 1 to 20 µM.

The period of the culture of hematopoietic precursor cells in the presence of the CDK8 inhibitor is not particularly limited. For example, the hematopoietic precursor cells can be cultured for 0.5 to 30 days, 1 to 20 days, or 2 to 10 days. The culture temperature is also not particularly limited. The culture temperature can be around 30 to 40°C, preferably around 37°C. In the present invention, the culture is preferably performed in a CO2-containing air atmosphere. The concentration of CO₂ is around 2 to 5%, preferably around 5%.

The culture method is not particularly limited in the present invention. For example, the cells may be separated by a method known in the art to be cultured by suspension culture or adherent culture. In the case of the adherent culture, the culture vessel may be coated to be used. The coculture with feeder cells or the like may be performed. The feeder cells for the coculture are preferably stromal cells. Specific examples include a bone marrow interstitial cell line, OP9 cells (available from the RIKEN BioResource Center) and MS5 cells. The feeder cells are preferably cells that express Delta-like 1 (Dll1) or Delta-like 4 (Dll4)constantly (NPLs 8 and 9). If the feeder cells are not used, the culture vessel may be coated with Dll1 or Dll4, or a fusion protein of Dll1 or Dll4 with Fc or the like to be used. The culture vessel is preferably used that is coated with 1 to 10 µg/mL Fc-DLL4 solution.

In the present invention, IL-2 and/or IL-33 is added to the medium in a preferable aspect, IL-2 and IL-33 is added in a more preferable aspect. Known IL-2 and/or IL-33 are usable. For example, IL-2 is available from BioLegend (589108) or PeproTech (200-02), and IL-33 is available from FUJIFILM Wako Pure Chemical Corporation (099-05611) or BioLegend (581806). The concentration of IL-2 can be suitably selected by those skilled in the art. The concentration thereof is preferably 1 to 10000 U/mL, more preferably 10 to 1000 U/mL, further preferably 100 to 300 U/mL. The concentration of IL-33 can also be suitably selected by those skilled in the art. The concentration thereof is preferably 0.1 to 10000 ng/mL, more preferably 1 to 1000 ng/mL, further preferably 10 to 100 ng/mL. In an embodiment, the medium for differentiation-inducing ILC2s may further contain one or more proteins selected from the group consisting of SCF, TPO (thrombopoietin), FLt3L, SDF-1α, and IL-7. For example, the concentrations of these are as follows. The concentration of SCF is 10 to 100 ng/mL. The concentration of TPO is 10 to 200 ng/mL. The concentration of FLt3L is 10 to 100 ng/mL. The concentration of SDF-1α is 100 to 500 ng/mL. The concentration of IL-7 is 10 to 100 ng/mL.

In the present culture step, the medium may be suitably replaced. The types and the concentrations of the additives contained in the medium may be suitably adjusted.

### Purposes (use) of group 2 innate lymphoid cells obtained by the present invention

The present invention includes the group 2 innate lymphoid cells (ILC2s) obtained by the present invention. In an embodiment, the present invention also includes the IL-10-producing ILC2s. These ILC2s is usable for various purposes without limitation. The ILC2s obtained by the present invention is usable not only for experiments in laboratories but also as therapeutic agents for diseases. Especially since the present invention enables obtaining IL-10-producing ILC2s, the ILC2s according to the present invention are effective, for example, in the treatment or the prevention of inflammatory diseases. In an embodiment, the present invention provides a therapeutic agent for inflammatory disease containing the IL-10-producing ILC2s obtained by the method of the present invention and a method for treating inflammatory disease including administering the IL-10-producing ILC2s to a subject.

Examples of the diseases to which the present invention is applied include inflammatory diseases such as ulcerative colitis and Crohn's disease; autoimmune diseases such as rheumatoid arthritis, systemic lupus erythematosus, type 1 diabetes, multiple sclerosis, pernicious anemia, pemphigus, and vasculitis; allergic diseases; cancer; acute respiratory distress syndrome (ARDS); and chronic obstructive pulmonary disease (COPD).

If the disease of a subject is treated using the ILC2s obtained by the present invention, the ILC2s according to the present invention are administered to the subject as the therapeutic agent. Although the method for administration to a subject is not particularly limited, for example, the ILC2s may be administered to a subject as a pharmaceutical composition containing a carrier or a vehicle to be commonly used for formulation and other additives. Examples of the method for administering the ILC2s to a subject include a method involving suspending the produced ILC2s in physiological saline or the like with a carrier or a vehicle for formulation and other additives, followed by transplantation directly into tissue of a subject and a method involving suspending the produced ILC2s in physiological saline or the like with a carrier or a vehicle for formulation and other additives to intravenously inject the suspension.

If the pharmaceutical composition containing the ILC2s according to the present invention is administered to a subject, the number of the ILC2s to be administered may be suitably adjusted by increasing or decreasing depending on the seriousness and the size of the affected part or the body of the patient.

Although the present invention will be described in further detail based on specific examples hereinafter, the present invention is not limited to the following specific examples. The concentration as mentioned herein means concentration by mass. The numerical ranges are described as a numerical range including the endpoints thereof unless otherwise specified.

### EXAMPLES

### Experiment 1: Differentiation induction from iPS cells to hematopoietic precursor cells (HPCs)

An ultra-low adhesion-treated six-well plate (Corning, 3471) was seeded with iPS cells at 3.0 × 10⁵ cells/well (Day 0), which iPS cells were cultured in iPS maintenance medium (Ajinomoto Co., Inc., StemFit AK02N) containing ROCK (Rho-associated coiled-coil forming kinase) inhibitor and GSK-3 (glycogen synthase kinase 3) inhibitor at 37°C in a 5% CO₂ atmosphere for one day to obtain embryoid bodies (EBs). In the present experiment, the cell line 1231A2 or Ff-I01s04, established in Center for iPS Cell Research and Application (CiRA), Kyoto University, was used as the iPS cells. Y-27632 (FUJIFILM Wako Pure Chemical Corporation, 036-24023 or 034-24024) was used as the ROCK inhibitor. CHIR99021 (Tocris, 4423) was used as the GSK-3 inhibitor. Each inhibitor was added to the iPS maintenance medium at 10 µM.

The iPS maintenance medium was subsequently replaced with embryonic body culture medium (EB medium). To the medium was added 50 ng/mL BMP4 (R&D, 314-BP), 50 ng/mL VEGF (R&D, 293-VE-500MG/CF), and 50 ng/mL FGF-2 (PeproTech, 100-18B) (Day 1). SB431542 (CAYMAN, 13031) was added at a final concentration of 6 µM on the next day (Day 2). The cells were further cultured for two days, followed by replacement with new EB medium and addition of 50 ng/mL SCF (R&D Systems, 255-SC-200 or FUJIFILM Wako Pure Chemical Corporation, 193-15513), 50 ng/mL VEGF, and 50 ng/mL FGF-2 (Day 4). The cells were successively cultured for three days, followed by replacement with new EB medium and addition of 50 ng/mL SCF, 50 ng/mL VEGF, 50 ng/mL FGF-2, 30 ng/mL TPO (PeproTech, 300-18 or FUJIFILM Wako Pure Chemical Corporation, 204-16474), and 10 ng/mL Flt3L (PeproTech, 300-19 or FUJIFILM Wako Pure Chemical Corporation, 067-05393) (Day7). After culture for a total of 9 to 11 days from Day 0, the culture solution containing the hematopoietic precursor cells was collected and passed through a 40 µm filter to be separated into single cells, followed by centrifugation under the conditions of 400 g, 4°C, and 5 minutes. After removal of the supernatant, the HPCs were suspended in cell cryopreservation solution (KAC Co., Ltd., TC Protector), followed by cryopreservation at -80°C (it was determined that the thus preserved cells were referred to as HPC stock cells).

The composition of the EB medium used in the present experiment is as follows.

### [EB medium]

- StemPro-34 SFM (trademark, Gibco, 10639011)
- 1% GlutaMAX (trademark, Gibco, 35050061)
- 1% Insulin-transferrin-selenium mixed reagent (ITS-G, Thermo Fisher Scientific, 41400045)
- 0.2% L-Glutamine-penicillin-streptomycin solution (Sigma Aldrich, G1146)
- 0.4 mM α-monothioglycerol (Sigma Aldrich, M1753)
- 50 µg/mL Ascorbic acid-2-phosphate (Sigma Aldrich, A8960)

### Experiment 2: Differentiation induction from iPS cell-derived HPCs to ILC2s (1)

The HPCs prepared from the iPS cells in Experiment 1 were cultured to be differentiation-induced into ILC2s. Specifically, 2500 to 20000 HPC stock cells prepared from the line 1231A2 were seeded in a 96-well plate (Corning, 3799 or Iwaki, 3860-096) coated with 2.5 µg/mL Fc-DLL4 and 2.5 µg/mL RetroNectin (TM) (Day 0) and cultured at 37°C in a 5% CO₂ atmosphere under the medium conditions shown below for 12 days to be differentiation-induced.

In the differentiation induction culture for 12 days, 100 µL of medium was added on Day 3, followed by medium replacement on Day 6 and Day 9. If the cells in each culture well are confluent after the medium replacement on Day 6 and Day 9, the cells in the well were suspended, 2-fold diluted, and reseeded in a new 96-well plate. In the cases of Conditions 2 to 4, IL-2 (BioLegend, 589108 or PeproTech, 200-02) was added to medium A at 200 U/mL. In the case of Condition 3, Senexin A (Selleck, S8520) was added to medium B at 3 µM as CDK8 inhibitor. In the case of Condition 4, CCT-251921 (MedChemExpress, HY-19984) was added to medium B at 100 nM as CDK8 inhibitor.

**[Table 1]**

| Table 1: Medium condition | | |
|---|---|---|
| Medium condition | Day 0-6 | Day 6-12 |
| Condition 1 (Comparative Example) | Medium A | Medium A |
| Condition 2 (Comparative Example) | Medium A + 200 U/mL IL-2 | Medium B |
| Condition 3 (Example) | Medium A + 200 U/mL IL-2 | Medium B + CDK8 inhibitor (Senexin A) |
| Condition 4 (Example) | Medium A + 200 U/mL IL-2 | Medium B + CDK8 inhibitor (CCT-251921) |

Cancer Sci. 2020 May; 111(5): 1478-1490 discloses that iPS cells were differentiation-induced into NK/ILC1-like cells by the following three steps:
- Step 1: A step of inducing hematopoietic precursor cells from iPS cells (for 14 days),
- Step 2: A step of inducing lymphoid precursor cells from the iPS cell-derived hematopoietic precursor cells on FcDLL4 (for 21 days); and
- Step 3: A step of producing and proliferating NK/ILC1-like cells.

It was confirmed in preliminary examination by the present inventors that a very small number of ILC1-like cells that produce IFN-γ or other cells are present on Day 12 in "Step 2". Various factors were therefore examined in view of the possibility that pluripotent ILC precursor cells having the capacity to differentiate into NK cells and all the ILC subsets appeared during this "Step 2". The "Condition 1" in this experiment corresponds to 12 days in the former half of "Step 2" in the above-mentioned method for differentiation-inducing NK/ILC1-like cells.

The media A and B used in this experiment were prepared as shown in the following tables.

**[Table 2]**

| Table 2: Preparation of medium A | |
|---|---|
| α-MEM (Invitrogen, 11900-024) | 42mL |
| FBS (fetal bovine serum) | 7.5mL |
| L-Glutamine-penicillin-streptomycin mixed solution | 0.5mL |
| Insulin-transferrin-selenium mixed reagent | 0.5mL |
| 2-Mercaptoethanol (Invitrogen, 21985-023) | Final concentration 55 µM |
| Ascorbic acid-2-phosphate | Final concentration 50 µg/mL |
| SCF | Final concentration 50 ng/mL |
| TPO | Final concentration 100 ng/mL |
| Flt3L | Final concentration 50 ng/mL |
| IL-7 (Peprotech, 200-07) | Final concentration 50 ng/mL |
| SDF-1α (Peprotech, 300-28A) | Final concentration 240 ng/mL |
| SB203580 (TOCRIS, 1202) | Final concentration 15 µM |

**[Table 3]**

| Table 3: Preparation of medium B | |
|---|---|
| α-MEM | 42mL |
| FBS (fetal bovine serum) | 7.5mL |
| L-Glutamine-penicillin-streptomycin mixed solution | 0.5mL |
| Insulin-transferrin-selenium mixed reagent | 0.5mL |
| 2-Mercaptoethanol | Final concentration 55 µM |
| Ascorbic acid-2-phosphate | Final concentration 50 µg/mL |
| IL-7 | Final concentration 50 ng/mL |
| SDF-1α | Final concentration 240 ng/mL |
| IL-2 | Final concentration 200 U/mL |
| IL-33 (FUJIFILM Wako Pure Chemical Corporation, 099-05611 or BioLegend, Inc., 581806) | Final concentration 20 ng/mL |

The differentiated cells cultured as described above were stimulated at 37°C in a 5% CO₂ atmosphere for 4 to 6 hours using the following:
- IL-1β (PeproTech, 200-01B), final concentration: 10 ng/mL
- IL-2, final concentration: 20 ng/mL
- IL-7, final concentration: 50 ng/mL
- IL-23 (BioLegend, 574106), final concentration: 20 ng/mL
- IL-25 (IL-17E) (PeproTech, 200-24), final concentration: 20 ng/mL
- eBioscience(TM) Cell Stimulation Cocktail (plus Protein transport inhibitor)

### (Thermo Fisher Scientific, 00-4975-03)

The stimulated cells were subsequently stained and analyzed with an LSRFortessa(TM) Cell Analyzer (BD Biosciences) by flow cytometry. The cells were stained with the following and analyzed by FlowJo(TM) (BD Biosciences).
- LIVE/DEAD(TM) Fixable Aqua Dead Cell Stain Kit (Thermo Fisher Scientific, L-34957)
- Anti-CD3 antibody (BioLegend, UCHT1)
- Anti-CD14 antibody (BioLegend, M5E2)
- Anti-CD16 antibody (BioLegend, 3G8)
- Anti-CD19 antibody (BioLegend, HIB19)
- Anti-CD20 antibody (BioLegend., 2H7)
- Anti-CD94 antibody (BD Pharmingen, HP-3D9)
- Anti-GATA3 antibody (Thermo Fisher Scientific, TWAJ)
- Anti-IL-13 antibody (BioLegend, JES-5A2)
- Anti-IL-10 antibody (BioLegend, JES-9D7)

Fig. 1-1 shows representative examples of the results of the flow cytometry. Fig. 1-2 shows the percentage of the number of lineage marker (CD3, CD14, CD16, CD19, CD20, and CD94)-negative GATA3-positive IL-13-positive cells in live cells (%ILC2) and the percentage of the number of lineage marker-negative IL-13-positive IL-10-positive cells in live cells (%IL-10+ ILC2).

In the case of "Condition 3", in which Senexin A as CDK8 inhibitor was added, the results of the flow cytometry confirmed that ILC2-like cell populations were induced that did not express lineage markers (CD3, CD14, CD16, CD19, CD20, and CD94), expressed the transcription factor GATA3, and had the capacity to produce IL-13. Some of the ILC2-like cells induced under "Condition 3" had the capacity to produce IL-10. Also in the case of "Condition 4", in which CCT-251921, having a structure different from the structure of Senexin A, was added as CDK8 inhibitor, it was confirmed that ILC2-like cell populations were induced in the same way as in the case of "Condition 3".

It was conceivable form these results that HPCs were able to be cultured in the presence of CDK8 inhibitor to be differentiation-induced into ILC2s.

### Experiment 3: Differentiation induction from iPS cell-derived HPCs to ILC2s (2)

In order to confirm the versatility of the method for differentiation induction of Experiment 2, it was ascertained in the present experiment that HPCs derived from a different iPS cell line (line Ff-I01s04) were differentiation-induced into ILC2s. Specifically, the HPC stock cells prepared from iPS cells (line Ff-I01s04) by the method described in Experiment 1 were cultured to be differentiation-induced into ILC2s by the method described in Experiment 2.

Fig. 2 shows representative examples of the results of the analysis by flow cytometry as described in Experiment 2. It was observed that, in the cases of "Condition 3" and "Condition 4", using CDK8 inhibitor, ILC2-like cells containing IL-10-producing cells were differentiation-induced from the HPCs derived from the line Ff-I01s04 as well as HPCs derived from the line 1231A2.

That is, it was able to be confirmed that ILC2s were able to be differentiation-induced even from both types of HPCs prepared from the different iPS cell lines, and the method for differentiation induction according to the present invention exhibited versatility.

### Experiment 4: Differentiation induction from iPS cell-derived HPCs to ILC2s (3)

The present experiment confirmed the differentiation induction into ILC2s using multiple CDK8 inhibitors having different structures. Specifically, the HPC stock cells prepared from iPS cells (line 1231A2) by the method described in Experiment 1 were cultured under Condition 2 of Experiment 2 with each of the CDK8 inhibitors (Compounds 1 to 4) added (as shown in the following table) to be differentiation-induced into ILC2s.

Fig. 3 shows the results of analysis by flow cytometry as described in Experiment 2. Also if any compound of Compounds 1 to 4 was added, the differentiation induction into ILC2-like cells containing IL-10-producing cells were observed in the same way as if Senexin A or CCT-251921 was added.

It was conceivable from these results that HPCs were able to be cultured in the presence of CDK8 inhibitor to be differentiation-induced into ILC2s.

**[Table 4]**

| Table 4: Medium condition | | |
|---|---|---|
| Medium condition | Day 0-6 | Day 6-12 |
| Condition 2 (Comparative Example) | Medium A + 200U/mL IL-2 | Medium B |
| Condition 5 (Example) | Medium A + 200U/mL IL-2 | Medium B + Compound 1 (1 µM MSC2530818, Selleck, S8387) |
| Condition 6 (Example) | Medium A + 200U/mL IL-2 | Medium B + Compound 2 (1 µM CCT251545, MedChemExpress HY-12681) |
| Condition 7 (Example) | Medium A + 200U/mL IL-2 | Medium B + Compound 3 (1 µM SEL120-34A, Selleck, S8840) |
| Condition 8 (Example) | Medium A + 200U/mL IL-2 | Medium B + Compound 4 (10 µM BRD6989, MedChemExpress HY-122586) |

### Experiment 5: Differentiation induction from human cord blood-derived CD34-positive HPCs to ILC2s

In order to ascertain the versatility of the method for differentiation induction in Experiment 2, the present experiment confirmed the differentiation induction from HPCs derived from the human body to ILC2s. Specifically, ILC2s were differentiation-induced by culturing purchased human cord blood-derived CD34-positive cells (Lonza, 2C-101) with the methods of Condition 1, Condition 2, and Condition 3 in Experiment 2. In the present experiment, the medium was also replaced with the same medium as on Days 6 to 12 on Day 12 and Day 15 of the culture, and the cells in each culture well were 2-fold diluted and reseeded in the case where the cells in the culture well were confluent.

Fig. 4 shows representative examples of the results of analysis by flow cytometry on Day 19 of the culture in the same way as on Day 12 of Experiment 2. It was observed that, in the cases of "Condition 3", using CDK8 inhibitor, ILC2-like cells containing IL-10-producing cells were differentiation-induced from the purchased human cord blood-derived HPCs as well as iPS cell-derived HPCs.

That is, it was able to be confirmed that the method for differentiation induction according to the present invention is applicable to not only iPS cell-derived HPCs but also HPCs separated from the human body.

### Experiment 6: Concentrations of cytokines in differentiation culture supernatant containing iPS cell-derived ILC2s

The culture supernatant of Day 12 of the culture was collected that was differentiation-induced under "Condition 1", "Condition 2", and "Condition 3" described in Experiment 2, followed by the quantification of concentrations of IL-10, IL-5, and amphiregulin in the culture supernatant with the following kits, respectively. Amphiregulin is a cytokine that plays a central role in the capacity of ILC2s to repair tissue. It is conceivable that ILC2s having the capacity to repair tissue were obtained in the case where amphiregulin increased in concentration.
- IL-10: Human IL-10 immunoassay kit (PerkinElmer, AL218C)
- IL-5: Human IL-5 immunoassay kit (PerkinElmer, AL267C)
- Amphiregulin: Human Amphiregulin Quantikine ELISA Kit (R&D Systems, DAR00)

IL-10 and IL-5 were measured with an Envision (Revvity). Amphiregulin was measured with a Powerscan4 (DS pharma biomedical Co., LTD.). As shown in Fig. 5, the enhancement of production of IL-10, IL-5, and amphiregulin in the culture supernatant was observed in the case of "Condition 3", in which the induction of ILC2s was confirmed in Experiment 2.

### Experiment 7: Suppressive effect of IL-10-producing ILC2s on production of TNFα by THP-1 cells

It was confirmed that the differentiated cells (cryopreserved at -80°C) on Day 12 of the culture induced under "Condition 1" and "Condition 3" described in Experiment 2 had the suppressive effect on the production of TNFα by THP-1 cells.

### 7-1. THP-1 cells

First, 1 × 10⁷ THP-1 cells (ATCC) were suspended in 10 mL of THP-1 growth medium, followed by addition of PMA (phorbol myristate acetate, Sigma Aldrich, P-1585) as a cell stimulation reagent at a final concentration of 2 µg/mL. The THP-1 cells were cultured in a 100-mm dish (Iwaki, 3020-100) at 37°C in a 5% CO₂ atmosphere for two days to be differentiation-induced into macrophages. The THP-1 cells were collected on Day 2 of the culture and centrifuged under the conditions of 300 g, room temperature, and five minutes. The supernatant was removed, followed by adding THP-1 growth medium so that the THP-1 cells were diluted to 1 × 10⁶ cells/mL.

### 7-2. Differentiated cells

The cryopreserved differentiated cells obtained under "Condition 1" and "Condition 3" of Experiment 2 were suspended in media shown in the following table in the same way as in Experiment 2 and cultured in a 96-well plate (Iwaki, 3860-096) at 37°C in a 5% CO₂ atmosphere for two days. After the culture, the differentiated cells obtained under "Condition 1" and "Condition 3" were collected and centrifuged under the conditions of 300 g, room temperature, and five minutes. The supernatant was removed, followed by adding ILC minimal essential medium so that the cells were diluted to 1 × 10⁵ cells/mL or 2 × 10⁴ cells/mL.

**[Table 5]**

| | |
|---|---|
| Condition 1 (Comparative Example) | Medium A |
| Condition 3 (Example) | Medium B + CDK8 inhibitor (3 µM Senexin A) |

### 7-3. ILC2-like cells' suppression of production of TNFα

Lipopolysaccharide (LPS) and IFN-γ were subsequently contacted with the THP-1 cells differentiated into macrophages, resulting in inflammation. The secreted amount of TNFα as an inflammatory cytokine was confirmed. Specifically, the following (a) to (c) were cultured in a 96-well plate with media for functional evaluation containing LPS and IFN-γ at 37°C in a 5% CO₂ atmosphere for two days.
(a) The differentiated cells obtained under "Condition 1" (5 × 10³ cells or 1 × 10³ cells) and the THP-1 cells (ATCC, 5 × 10⁴ cells)
(b) The differentiated cells obtained under "Condition 3" (5 × 10³ cells or 1 × 10³ cells) and the THP-1 cells (ATCC, 5 × 10⁴ cells)
(c) only the THP-1 cells (ATCC, 5 × 10⁴ cells) (THP-1 only)

The culture supernatants were collected on Day 2 of the culture and measured for the concentration of TNFα with an immunoassay kit (Human TNFα immunoassay kit, PerkinElmer Japan G.K., AL208C). The concentrations of TNFα were measured with an Envision in accordance with the protocol attached to the kit.

Fig. 6 shows the results of measuring the concentrations of TNFα in the culture supernatants. As is clear from these results of the measurement, the differentiated cells under "Condition 3" suppressed the production of TNFα in the THP-1 cells.

The compositions of the THP-1 growth medium, ILC minimum essential medium, and medium for functional evaluation of THP-1 cells were as follows.

### [THP-1 growth medium]

- RPMI medium (FUJIFILM Wako Pure Chemical Corporation, 189-02025)
- 10% FBS
- 1% L-Glutamine-penicillin-streptomycin mixed solution

### [ILC minimum essential medium]

- α-MEM
- 15% FBS
- 1% L-Glutamine-penicillin-streptomycin mixed solution

### [Medium for functional evaluation]

- 44% RPMI medium
- 42% α-MEM
- 12% FBS
- 1% L-Glutamine-penicillin-streptomycin mixed solution
- 1% Insulin-transferrin-selenium mixed reagent
- 55 µM 2-Mercaptoethanol
- 50 µg/mL Ascorbic acid-2-phosphate
- 50 ng/mL IL-7
- 240 ng/mL SDF-1α
- 200 U/mL IL-2
- 20 ng/mL IL-33
- 100 ng/mL LPS (Sigma Aldrich, L6511)
- 20 ng/mL IFN-γ (BioLegend, 570204)

### Experiment 8: Suppressive effect of CD25 and CD30-expressing cells in IL-10-producing ILC2s on the production of TNFα by THP-1 cells

Examined were the suppressive effects of a CD25+CD30+ population and a CD25-CD30- population in the differentiated cells (cryopreserved at -80°C) on Day 12 of the culture induced under "Condition 3" described in Experiment 2 on the production of TNFα by THP-1 cells.

### 8-1. THP-1 cells

First, 1 × 10⁷ THP-1 cells (ATCC) were suspended in 15 mL of THP-1 growth medium, followed by addition of PMA as a cell stimulation reagent at a final concentration of 2 µg/mL. The THP-1 cells were cultured in a 100-mm dish at 37°C in a 5% CO₂ atmosphere for two days to be differentiation-induced into macrophages. The THP-1 cells were collected on Day 2 of the culture and centrifuged under the conditions of 300 g, room temperature, and five minutes. The supernatant was removed, followed by adding THP-1 growth medium so that the THP-1 cells were diluted to 1 × 10⁶ cells/mL.

### 8-2. ILC2s, differentiated cells

The cryopreserved differentiated cells obtained under "Condition 3" of Experiment 2 were suspended in medium shown in the following table in the same way as in Experiment 2 and cultured in a 96-well plate at 37°C in a 5% CO₂ atmosphere for two days. After the culture, the differentiated cells obtained under "Condition 3" were collected and centrifuged under the conditions of 300 g, room temperature, and five minutes. The supernatant was removed, followed by adding 100 µL of PBS(-), 4 µL of anti-CD25 antibody (Miltenyi, REA570), 40 µL of anti-CD30 antibody (BD Biosciences, BerH8), and 1 µL of LIVE/DEAD (TM) Fixable Aqua Dead Cell Stain Kit. The mixture was left to stand on ice for 20 minutes. The cells were then washed with PBS(-) twice. The CD25-CD30- fraction and the CD25+CD30+ fraction were separately collected with FACS Aria4 (BD Biosciences). Cells in each fraction were prepared with ILC minimal essential medium to 2 × 10⁵ cells/mL, 1 × 10⁵ cells/mL, or 2 × 10⁴ cells/mL.

**[Table 6]**

| | |
|---|---|
| Condition 3 | Medium B + CDK8 inhibitor (3µM Senexin A) |

### 8-3. ILC2-like cells' suppression of production of TNFα

Lipopolysaccharide (LPS) and IFN-γ were subsequently contacted with the THP-1 cells differentiated into macrophages, resulting in inflammation. The secreted amount of TNFα as an inflammatory cytokine was confirmed. Specifically, the following (a) to (c) were cultured in a 96-well plate with media for functional evaluation of THP1 containing LPS and IFN-γ at 37°C in a 5% CO₂ atmosphere for two days.
(a) The CD25-CD30- cells obtained under "Condition 3" (1 × 10⁴ cells, 5 × 10³ cells, or 1 × 10³ cells) and the THP-1 cells (ATCC, 5 × 10⁴ cells)
(b) The CD25+CD30+ cells obtained under "Condition 3" (1 × 10⁴ cells, 5 × 10³ cells, or 1 × 10³ cells) and the THP-1 cells (ATCC, 5 × 10⁴ cells)
(c) only the THP-1 cells (ATCC, 5 × 10⁴ cells) (THP-1 only)

The culture supernatants were collected on Day 2 of the culture and measured for the concentration of TNFα and the concentration of IL-10 with an immunoassay kit. The concentrations of the cytokines were measured with an Envision in accordance with the protocol attached to the kit.

The top of Fig. 7 shows the results of measuring concentrations of TNFα in the culture supernatants. As is clear from these results, while the CD25+CD30+ cells of "Condition 3" suppressed the production of TNFα by the THP-1 cells, the CD25-CD30- cells did not suppress the production of TNFα. The bottom of Fig. 7 shows the results of measuring the concentrations of IL-10 in the culture supernatants. While the CD25+CD30+ cells produced IL-10, it was not observed that the CD25-CD30- cells produced IL-10.

### Experiment 9: Suppressive effect of IL-10-producing ILC2s on the production of TNFα by PBMC-derived macrophages

Examined was the suppressive effect of the differentiated cells (cryopreserved at - 80°C) on Day 12 of the culture induced under "Condition 3" described in Experiment 2 on the production of TNFα by PBMC-derived macrophages of IL-10-producing ILC2s.

### 9.1 PBMC-derived macrophages

The PBMC stock thawed at 37°C was suspended in macrophage differentiation medium containing 20 U/mL DNase (Merck KGaA, D5025) and centrifuged under the conditions of 300 g, room temperature, and five minutes. The supernatant was removed, followed by addition of 1 mL of macrophage differentiation medium. CD14-positive cells were separately collected from the PBMC suspension with CD14 microbeads (Miltenyi, 130-050-201). The obtained CD14-positive cells were centrifuged under the conditions of 300 g, room temperature, and five minutes and suspended in 3 mL of macrophage differentiation medium containing 100 ng/mL GM-CSF (Peprotech, 300-03). Then, 1 mL thereof were added to each of the three wells in a 24-well plate (Iwaki, 3820-024), followed by culture at 37°C in a 5% CO₂ atmosphere. Then, 800 µL of the supernatant was removed on each of Day 3 and Day 6 after the beginning of the culture, followed by addition of 1 mL of macrophage differentiation medium containing 100 ng/mL GM-CSF. The cell supernatant was removed on Day 8 after the beginning of the culture. Then, 300 µL of PBS was added, followed by removing the supernatant again. Subsequently, 200 µL of Accutase (Innovative Cell Technologies, Inc., AT104) was added, followed by leaving the mixture to stand at 37°C for 10 minutes. Then, 800 µL of macrophage differentiation medium was added, followed by collection of the cells. The mixture was centrifuged under the conditions of 300 g, room temperature, and five minutes, followed by adding macrophage differentiation medium so that the cells were diluted to 4 × 10⁵ cells/mL.

### 9-2. ILC2s, differentiated cells

The cryopreserved differentiated cells obtained under "Condition 1" and "Condition 3" of Experiment 2 were suspended in the media shown in the following table in the same way as in Experiment 2, followed by culture in a 96-well plate at 37°C in a 5% CO₂ atmosphere for two days. After the culture, the differentiated cells obtained under "Condition 1" and "Condition 3" were collected and centrifuged under the conditions of 300 g, room temperature, and five minutes. The supernatant was removed, followed by adding ILC minimum essential medium so that the cells were diluted to 4 × 10⁵ cells/mL or 8 × 10⁴ cells/mL.

**[Table 7]**

| | |
|---|---|
| Condition 1 (Comparative Example) | Medium A |
| Condition 3 (Example) | Medium B + CDK8 inhibitor (3 µM Senexin A) |

### 9.3. ILC2-like cells' suppression of production of TNFα

Lipopolysaccharide (LPS) and IFN-γ were subsequently contacted with the PBMC-derived macrophages, resulting in inflammation. The secreted amount of TNFα as an inflammatory cytokine was confirmed. Specifically, the following (a) to (c) were cultured in a 96-well plate with medium for functional evaluation of PBMC-derived macrophages containing LPS and IFN-γ at 37°C in a 5% CO₂ atmosphere for two days.
(a) The differentiated cells obtained under "Condition 1" (2 × 10⁴ cells or 4 × 10³ cells) and the PBMC-derived macrophages (2 × 10⁴ cells)
(b) The differentiated cells obtained under "Condition 3" (2 × 10⁴ cells or 4 × 10³ cells) and the PBMC-derived macrophages (2 × 10⁴ cells)
(c) only the PBMC-derived macrophages (2 × 10⁴ cells)

The culture supernatants were collected on Day 2 of the culture and measured for the concentration of TNFα with the immunoassay kit. The concentrations of TNFα were measured with an Envision in accordance with the protocol attached to the kit.

Fig. 8 shows the results of measuring the concentration of TNFα in the culture supernatant. As is clear from the results of the measurement, the differentiated cells of "Condition 3" suppressed the production of TNFα by the PBMC-derived macrophages.

### [Macrophage differentiation medium]

- RPMI medium
- 10% FBS
- 1% L-Glutamine-penicillin-streptomycin mixed solution
- 1% Sodium pyruvate solution (NACALAI TESQUE, INC., 06977-034)
- 1% MEM non-essential amino acid solution (FUJIFILM Wako Pure Chemical Corporation, 139-15651)

### [Medium for functional evaluation of PBMC-derived macrophages]

- 44% RPMI medium
- 41% α-MEM
- 12% FBS
- 1% L-Glutamine-penicillin-streptomycin mixed solution
- 1% Insulin-transferrin-selenium mixed reagent
- 0.5% Sodium pyruvate solution
- 0.5% MEM non-essential amino acid solution

- 55 µM 2-Mercaptoethanol
   - 50 µg/mL Ascorbic acid-2-phosphate
   - 50 ng/mL IL-7
   - 240 ng/mL SDF-1α
   - 200 U/mL IL-2
   - 20 ng/mL IL-33
   - 100 ng/mL LPS
   - 20 ng/mL IFN-γ

### INDUSTRIAL APPLICABILITY

As detailed above, the present invention provides a method involving differentiation-inducing ILC2s or IL-10-producing ILC2s from hematopoietic precursor cells derived from pluripotent stem cells to produce the ILC2s or the IL-10-producing ILC2s. The ILC2s or the IL-10-producing ILC2s acquired by the present method are usable in pharmaceutical development.

## Claims

1. A method for producing group 2 innate lymphoid cells, comprising a step of culturing hematopoietic precursor cells in the presence of CDK8 inhibitor to obtain group 2 innate lymphoid cells.

2. The method according to claim 1, wherein the group 2 innate lymphoid cells comprise group 2 innate lymphoid cells that produce IL-10.

3. The method according to claim 1 or 2 to be performed *in vitro.*

4. The method according to claim 1 or 2, wherein the group 2 innate lymphoid cells are obtained by culturing hematopoietic precursor cells in the presence of CDK8 inhibitor, IL-2, and IL-33.

5. The method according to claim 1 or 2, wherein the CDK8 inhibitor comprises Senexin A, CCT-251921, MSC2530818, CCT251545, SEL120-34A, or BRD6989.

6. The method according to claim 1 or 2, wherein the hematopoietic precursor cells are differentiated from human induced pluripotent stem cells (human iPS cells).

7. The method according to claim 1 or 2, wherein the hematopoietic precursor cells are derived from the human body.

8. The method according to claim 1 or 2, further comprising a step of inducing differentiation from pluripotent stem cells to the hematopoietic precursor cells.

9. The method according to claim 8, wherein the pluripotent stem cells are induced pluripotent stem cells (iPS cells).

10. The method according to claim 8, wherein the pluripotent stem cells are human iPS cells.
